# EUROPÄISCHE PATENTANMELDUNG

(11) **EP 3 838 412 A1**
(43) Veröffentlichungstag der Anmeldung: **23.06.2021**
(21) Anmeldenummer: 19218747.4
(22) Anmeldetag: 20.12.2019
(51) Int. Cl.: B01L 3/02, A61M 37/00, G01F 15/00, G01F 13/00

(54) **VORRICHTUNG UND VERFAHREN ZUM DOSIERTEN ABGEBEN EINER MIKROFLUIDISCHEN FLUIDMENGE IM PIKO- UND MIKROLITERBEREICH UND HANDGERÄT ZUM LOKALEN AUFSTECHEN EINER MENSCHLICHEN ODER EINER TIERISCHEN HAUT**

(71) Anmelder: MT.DERM GmbH, 14167 Berlin (DE)
(72) Erfinder: Scherkowski, Dirk, 12489 Berlin (DE)
(74) Vertreter: Bittner, Thomas L.

(57) **Zusammenfassung**

Die Erfindung betrifft eine Vorrichtung und ein Verfahren zum dosierten Abgeben einer mikrofluidischen Fluidmenge im Piko- und Mikroliterbereich, Über eine Ausbringleitung (3) werden Fluidmengen eines Fluids aus einem Reservoir (1) dosiert ausgebracht. Es ist eine Druckeinrichtung (2) bereitgestellt, um das Fluid zum Ausstoßen über die Ausbringleitung (3) mit einem Ausbringdruck zu beaufschlagen. Das Ausstoßen wird mittels einer Ventileinrichtung gesteuert, welche ein erstes Ventil (4) vom Typ Schließer mit einer minimalen Schließzeit und ein zweites Ventil (5) vom Typ Öffner mit einer minimalen Öffnungszeit aufweist. Mittels einer Steuereinrichtung (6), werden Steuersignale zum jeweiligen Betätigen des ersten Ventils (4) und des zweiten Ventils (5) bereitgestellt, derart, dass mittels Betätigen des ersten und des zweiten Ventils (4, 5) für die Ausbringleitung (3) wenigstens einer der folgenden Betriebsmodi zum dosierten Abgeben der Fluidmenge ausführbar ist: eine verkürzte minimale Öffnungszeit zum Freigeben der Ausbringleitung (3) für den Fluidstrom, welche kürzer als die minimale Öffnungszeit des zweiten Ventils (5) ist; und eine verkürzte minimale Schließzeit zum Schließen der Ausbringleitung (3) für den Fluidstrom, welche kürzer als die minimale Schließzeit des ersten Ventils (4) ist. Des Weiteren ist ein Handgerät zum lokalen Aufstechen einer menschlichen oder einer tierischen Haut offenbart.

## Beschreibung

Die Erfindung betrifft eine Vorrichtung sowie ein Verfahren zum dosierten Abgeben einer mikrofluidischen Fluidmenge im Piko- und Mikroliterbereich und ein Handgerät zum lokalen Aufstechen einer menschlichen oder einer tierischen Haut.

### Hintergrund

In Verbindung mit verschiedenen Anwendungen besteht Bedarf, Fluidmengen aus einem Reservoir wiederholt dosiert bereitzustellen und abzugeben, zum Beispiel in Form Fluidpulsen. Dieses ist beispielsweise der Fall bei Handgeräten zum lokalen Aufstechen einer menschlichen oder einer tierischen Haut, sei es im Zusammenhang mit dem Tätowieren oder dem Einbringen eines medizinischen oder eines kosmetischen Wirkstoffes in die Haut. Mit Hilfe einer Stecheinrichtung des Handgeräts wird die Haut lokal aufgestochen. Zeitlich koordiniert hierzu soll eine Fluidmenge des in die Haut einzubringenden Stoffes bereitgestellt werden. Aber auch in anderen Anwendungen, zum Beispiel im Bereich der Mikrofluidik, ist es häufig notwendig, dosierte Fluidmengen wiederholt bereitzustellen.

Bei einer Vorrichtung, welche über ein Reservoir mit einem auszubringenden Fluid verfügt, kann in einer Ausbringleitung, die mit dem Fluidreservoir in Verbindung steht, ein Ventil benutzt werden, um die Ausbringleitung für die Fluidabgabe zu öffnen und zu schließen. Derartige Ventile verfügen üblicherweise über eine Ventilcharakteristik, die das Verhalten des Ventils im Betrieb charakterisiert. Hierzu gehört das zeitliche Verhalten betreffend das Öffnen und das Schließen des Ventils. Die Abgabe einer dosierten Fluidmenge ist hierbei im Fall eines Ventils vom Typ Öffner auf die minimale Öffnungszeit des verwendeten Ventils beschränkt. Umgekehrt ist die minimale Zeit, für welche die Ausbringleitung für den Fluidstrom geschlossen werden kann, durch die minimale Schließzeit eines Ventils vom Typ Schließer beschränkt, bei dem das Ventil in Ruhestellung offen und in Arbeitsstellung geschlossen ist.

### Zusammenfassung

Aufgabe der Erfindung ist es, eine Vorrichtung und ein Verfahren zum dosierten Abgeben einer mikrofluidischen Fluidmenge im Piko- und Mikroliterbereich sowie ein verbessertes Handgerät zum lokalen Aufstechen einer menschlichen oder einer tierischen Haut anzugeben, bei denen die Möglichkeiten für eine Variation des dosierten Abgebens einer Fluidmenge erweitert sind, insbesondere zum verkürzten Öffnen und Schließen einer Ausbringleitung, durch die die Fluidmenge ausgebracht wird.

Zur Lösung sind eine Vorrichtung und ein Verfahren zum dosierten Abgeben einer Fluidmenge nach den unabhängigen Ansprüchen 1 und 7 geschaffen. Weiterhin ist ein Handgerät zum lokalen Aufstechen einer menschlichen oder einer tierischen Haut nach dem nebengeordneten Anspruch 8 vorgesehen. Ausgestaltungen sind Gegenstand von abhängigen Unteransprüchen.

Nach einem Aspekt ist eine Vorrichtung zum dosierten Abgeben einer mikrofluidischen Fluidmenge im Piko- und Mikroliterbereich geschaffen, die Folgendes aufweist: ein Reservoir, welches eingerichtet ist, ein Fluid aufzunehmen; eine Ausbringleitung, die mit dem Reservoir in Fluidverbindung steht und durch die eine Fluidmenge des Fluids aus dem Reservoir ausgebracht werden kann; und eine Druckeinrichtung, die eingerichtet ist, das Fluid mit einem Ausbringdruck zu beaufschlagen, derart, dass die Fluidmengen über die Ausbringleitung ausgestoßen werden. Weiterhin weist die Vorrichtung eine Ventileinrichtung mit einem ersten Ventil vom Typ Schließer in der Ausbringleitung auf, welches in einer Ruhestellung offen ist, um die Ausbringleitung für einen Fluidstrom freizugeben, und in einer Arbeitsstellung geschlossen ist, um die Ausbringleitung für einen Fluidstrom zu schließen, wobei das erste Ventil als Ventilcharakteristik eine minimale Schließzeit aufweist. Die Ventileinrichtung verfügt in der Ausbringleitung weiterhin über ein zweites Ventil vom Typ Öffner, welches in Ruhestellung geschlossen ist, um die Ausbringleitung für den Fluidstrom zu schließen, und in einer Arbeitsstellung offen ist, um die Ausbringleitung für den Fluidstrom freizugeben, wobei das zweite Ventil als Ventilcharakteristik eine minimale Öffnungszeit aufweist. Bei der Vorrichtung ist eine Steuereinrichtung vorgesehen, welche mit dem ersten und dem zweiten Ventil verbunden und eingerichtet ist, erste Steuersignale zum Betätigen des ersten Ventils und zweite Steuersignale zum Betätigen des zweiten Ventils bereitzustellen, derart, dass mittels Betätigen des ersten und des zweiten Ventils für die Ausbringleitung wenigstens einer der folgenden Betriebsmodi zum dosierten Abgeben der Fluidmenge ausführbar ist: eine verkürzte minimale Öffnungszeit zum Freigeben der Ausbringleitung für den Fluidstrom, welche kürzer als die minimale Öffnungszeit des zweiten Ventils ist, und eine verkürzte minimale Schließzeit zum Schließen der Ausbringleitung für den Fluidstrom, welche kürzer als die minimale Schließzeit des ersten Ventils ist.

Nach einem weiteren Aspekt ist ein Verfahren zum dosierten Abgeben einer mikrofluidischen Fluidmenge im Piko- und Mikroliterbereich mit folgenden Schritten geschaffen: Bereitstellen eines abzugebenden Fluids in einem Reservoir; einer Ausbringleitung, die mit dem Reservoir in Fluidverbindung steht und durch die eine Fluidmenge des Fluids aus dem Reservoir ausgebracht werden kann; Beaufschlagen des Fluids mit einem Ausbringdruck mittels einer Druckeinrichtung, derart, dass die Fluidmenge über die Ausbringleitung ausgestoßen wird; und Steuern des Ausstoßens der Fluidmenge mittels einer Ventileinrichtung und einer hiermit verbundenen Steuereinrichtung. Die Ventileinrichtung weist Folgendes auf: ein erstes Ventil vom Typ Schließer in der Ausbringleitung, welches in einer Ruhestellung offen ist, um die Ausbringleitung für einen Fluidstrom freizugeben, und in einer Arbeitsstellung geschlossen ist, um die Ausbringleitung für einen Fluidstrom zu schließen, wobei das erste Ventil als Ventilcharakteristik eine minimale Schließzeit aufweist; und ein zweites Ventil vom Typ Öffner in der Ausbringleitung, welches in einer Ruhestellung geschlossen ist, um die Ausbringleitung für den Fluidstrom zu schließen, und in einer Arbeitsstellung offen ist, um die Ausbringleitung für den Fluidstrom freizugeben, wobei das zweite Ventil als Ventilcharakteristik eine minimale Öffnungszeit aufweist. Die Steuereinrichtung stellt erste Steuersignale zum Betätigen des ersten Ventils und zweite Steuersignale zum Betätigen des zweiten Ventils bereit, derart, dass mittels Betätigen des ersten und des zweiten Ventils für die Ausbringleitung wenigstens einer der folgenden Betriebsmodi zum dosierten Abgeben der Fluidmenge ausgeführt wird: eine verkürzte minimale Öffnungszeit zum Freigeben der Ausbringleitung für den Fluidstrom, weiche kürzer als die minimale Öffnungszeit des zweiten Ventils ist; und eine verkürzte minimale Schließzeit zum Schließen der Ausbringleitung für den Fluidstrom, welche kürzer als die minimale Schließzeit des ersten Ventils ist.

Nach einem weiteren Aspekt ist ein Handgerät zum lokalen Aufstechen einer menschlichen oder einer tierischen Haut mit einer solchen Vorrichtung zum dosierten Abgeben einer mikrofluidischen Fluidmenge im Piko- und Mikroliterbereich vorgesehen. Beispielsweise kann es sich um ein Handgerät zum Tätowieren oder für Permanent-Makeup handeln.

Die Kombination des ersten und des zweiten Ventils vom Typ Schließer / Öffner in der Ausbringleitung ermöglicht es, dass Ausbringen der dosierten Fluidmenge, zum Beispiel in Form von Fluidpulsen, mittels Betätigen beider Ventile zu steuern. Hierdurch wird die Variabilität für das Steuern zum Öffnen und zum Schließen der Ausbringleitung im Vergleich zum Vorsehen eines einzelnen Ventils mit einer festen Ventilcharakteristik verbessert. Die Kombination der Ventile ermöglicht eine verbesserte Anpassung der Betriebsmodi an unterschiedliche Anwendungsfälle zum dosierten Abgeben der Fluidmenge. Eine verkürzte minimale Öffnungszeit und / oder eine verkürzte minimale Schließzeit sind bereitgestellt. Hierdurch ist es ermöglicht, im Vergleich mit dem bekannten Betrieb mit nur einem Ventil, sei es vom Typ Schließer oder vom Typ Öffner, verkürzte Öffnungs- / Schließzeiten für die Ausbringleitung bereitzustellen, so dass verkürzte Fluidimpulse ermöglicht sind.

Es ist so ermöglicht, mikrofluidische Mengen des Fluids im Piko- und Mikroliterbereich (Gebiet der Mikrofluidik) bereitzustellen und abzugeben. Wenn gewünscht, können mit Hilfe der Anordnung von Ventilen auch größere Fluidmengen abgegeben werden, insbesondere dadurch, dass einfach die Öffnungszeit vergrößert wird, so dass größere Fluidmengen ausgegeben werden, zum Beispiel bis hin zu Millilitern. Die vorgeschlagene Technik ermöglicht es jedoch, insbesondere die mikrofluidischen Mengen des Fluids zu dosieren, wahlweise auch wiederholt aufeinander.

Neben der Kombination des ersten und des zweiten Ventils können ein oder mehrere weitere Ventile in der Ausbringleitung angeordnet sein. Das Betätigen des einen oder der mehreren weiteren Ventile zum Öffnen und zum Schließen kann von der Steuereinrichtung in Kombination mit dem ersten und dem zweiten Ventil koordiniert erfolgen.

In der Ausbringleitung kann in Bezug auf die Druckeinrichtung entweder das erste oder das zweite Ventil nächstliegend angeordnet sein.

Die Druckeinrichtung kann in einem Ausführungsbeispiel dadurch gebildet sein, dass die Fluidmenge allein aufgrund der Wirkung der auf sie wirkenden Schwerkraft mit dem Ausbringdruck (statischer Druck) beaufschlagt ist.

Die Steuereinrichtung kann weiterhin eingerichtet sein, die ersten Steuersignale zum Betätigen des ersten Ventils und die zweiten Steuersignale zum Betätigen des zweiten Ventils derart bereitzustellen, dass die verkürzte minimale Öffnungszeit zum Freigeben der Ausbringleitung für den Fluidstrom kürzer als die minimale Schließzeit des ersten Ventils ist. Bei dieser Ausführungsform ist die verkürzte minimale Öffnungszeit sowohl kürzer als die minimale Öffnungszeit des zweiten Ventils als auch kürzer als die minimale Schließzeit des ersten Ventils.

Die Steuereinrichtung kann eingerichtet sein, die ersten Steuersignale zum Betätigen des ersten Ventils und die zweiten Steuersignale zum Betätigen des zweiten Ventils derart bereitzustellen, dass die verkürzte minimale Schließzeit zum Schließen der Ausbringleitung für den Fluidstrom kürzer als die minimale Öffnungszeit des zweiten Ventils ist. Bei dieser Ausführungsform ist die verkürzte minimale Schließzeit zum Schließen der Ausbringleitung sowohl kürzer als die minimale Schließzeit des ersten Ventils als auch kürzer als die minimale Öffnungszeit des zweiten Ventils.

Die ersten und die zweiten Steuersignale können eingerichtet sein, wenigstens einen der folgenden Betriebsmodi mit einer Wiederholfrequenz bereitzustellen: wiederholtes Öffnen der Ausbringleitung für den Fluidstrom mit der verkürzten minimalen Öffnungszeit und wiederholtes Schließen der Ausbringleitung für den Fluidstrom mit der verkürzten minimalen Schließzeit. Bei dieser Ausführungsform werden Fluidimpulse oder -mengen einer pulsierten Betriebsart mit der Wiederholungsfrequenz bereitgestellt und abgegeben. Die Wiederholungsfrequenz kann beispielsweise etwa 0,1 Hz bis etwa 100 Hz betragen, alternativ etwa 0,1 Hz bis etwa 15 Hz.

Das erste Ventil und das zweite Ventil können in der Ausbringleitung einer Ventilreihenschaltung entsprechend angeordnet sein. Eine Reihenschaltung ist geeignet, möglichst genau dosierte, kleinste Fluidmengen abzugeben, zum Beispiel bei hoher Wiederholungsrate.

Das erste Ventil und das zweite Ventil können in der Ausbringleitung einer Ventilparallelschaltung entsprechend angeordnet sein. Die Parallelschaltung ist geeignet, besonders kurze Dosierunterbrechungen in einem sonst kontinuierlichen Fluidstrom zu erzeugen und so genau dosierte, kleinste Fluidunterbrechungen mit höchster Wiederholgenauigkeit und bei hoher Wiederholrate zu liefern.

Das erste und das zweite Ventil können in einem gemeinsamen Ventilgehäuse angeordnet sein. Insbesondere kann vorgesehen sein, die Anordnung so zu gestalten, dass ein gemeinsamer Fluidkontrollteil gebildet ist, an dem sowohl ein Öffner-Aktor, als auch ein Schließ-Aktor angreifen.

Die vorgeschlagene Technologie kann zum Beispiel für den Aufbau einer sogenannten Fluidleiterplatte in Lab-on-a-Chip-Konzepten und / oder bei einem Schaltelement in Fluidrechnern angewendet werden.

In Verbindung mit dem Handgerät kann vorgesehen sein, dass dieses ein Antriebsmodul und ein hieran gekoppeltes Stechmodul aufweist, sei es lösbar oder nicht lösbar koppelnd. Mithilfe einer Antriebseinrichtung im Antriebsmodul wird eine nicht-manuell (maschinell) erzeugte Antriebskraft oder -bewegung für eine im Stechmodul angeordnete Stecheinrichtung bereitgestellt, beispielsweise unter Verwendung eines Elektromotors. Das Stechmodul kann ein Einwegmodul sein. Ausführungsformen für solche Handgeräte zum lokalen Aufstechen einer Haut sind als solche in verschiedene Ausführungsformen bekannt.

Alternativ kann eine manuell betätigbare Stecheinrichtung vorgesehen sein. Um koordiniert zur manuell ausgelösten Stechbewegung die Abgabe oder Dosierung der Fluidmenge zu steuern, wird mittels einer Sensoreinrichtung die Stechbewegung detektiert. Zum Beispiel kann eine Sensoreinrichtung vorgesehen sein, mit der eine Bewegung einer Stechnadel erfasst wird. Alternativ oder ergänzend kann ein Kippsensor eine Kippbewegung erfassen, die die Stechbewegung anzeigt. In Abhängigkeit hiervon wird dann die Dosierung der mikrofluidischen Menge des Fluids bewirkt, insbesondere mittels Betätigens der Dosiereinrichtung.

In einer Ausgestaltung kann so eine Vorrichtung zum Injizieren oder Applizieren einer Substanz in eine menschliche oder eine tierische Haut gebildet sein. Die Vorrichtung kann eingerichtet sein für Lab-on-Chip-Anwendungen oder Fluiddosierungen im Medizinanalyse-Gerätbereich. Eine Ausgestaltung für die Ventilunion / -fusion kann vorgesehen sein, insbesondere in Verbindung mit Miniaturventilen.

Die mittels der vorgeschlagenen Technologie geschaffene Möglichkeit, ein Fluid in genau dosierter Menge wiederholgenau präzise und repetierend zu dosieren, insbesondere Fluidmengen im Mikro- bis Pikoliterbereich, kann in Anwendungsbereichen angewendet werden, zum Beispiel auf dem Gebiet von Sub-Miniaturventilen. Die Vorrichtung zum dosierten Abgeben der Fluidmenge ist dann als ein Sub-Miniaturventil ausgeführt. Solche Sub-Miniaturventile können mit einer Aktorik aus sogenannten Formgedächtnislegierungen (FGL) aufgebaut sein, beispielsweise mit Aktorteilen aus nur einem Draht- oder Folienbauteil. Diese verändern nach entsprechender Vorbehandlung und Legierungswahl bei Erwärmung gewünscht ihre Form, beispielsweise ein Übergang von einer ersten Form in eine zweite Form. Eine solche Erwärmung lässt sich beispielsweise mittels eines elektrischen Stromflusses erhalten, der dem metallischen FGL-Material als elektrisches Steuersignal aufgeprägt und über die Verlustleistung am elektrischen Widerstand des Materials umgesetzt wird. Bei Unterbrechung des elektrischen Steuerstroms kühlt sich der Aktorteil wieder ab und nimmt seine erste Form an. Mit Hilfe eines kontrolliert stärkeren Stromsignals lässt sich zwar die Erwärmung und damit der Formübergang in der Richtung Ruhelage zu Arbeitslage, also im Beispiel von der ersten Form in die zweite Form, forcieren, um eine kurze Anstiegszeit zu erhalten, der gegensätzliche Übergang erfolgt dann aber passiv über Abkühlvorgänge deutlich langsamer, wodurch die Abfallzeit nachteilig verlängert. Typische Sub-Miniaturventil-Schaltzeiten können beispielweise im Bereich von etwa 60 ms bis etwa 80 ms für das Öffnen und das Schließen symmetrisch liegen, forciert bei etwa 10 ms in einer ersten Richtung, wobei die Rückkehr in die Ausgangslage dann zum Beispiel etwa 120 ms oder mehr dauern kann.

In einer Ausführung ist bei der Vorrichtung zum dosierten Abgeben des Fluids vorgesehen, dass das erste und das zweite Ventil als ein Ventil mit einer Aktorik aus einer Formgedächtnislegierung aufgebaut ist, insbesondere ein Sub-Miniaturventil.

Die vorangehend im Zusammenhang mit der mikrofluidischen Vorrichtung zum dosierten Abgeben der mikrofluidischen Menge des Fluids erläuterten Ausgestaltungen können in Verbindung mit dem mikrofluidischen Verfahren zum dosierten Abgeben und / oder dem Handgerät entsprechend vorgesehen sein.

### Beschreibung von Ausführungsbeispielen

Im Folgenden werden weitere Ausführungsbeispiele unter Bezugnahme auf Figuren einer Zeichnung näher erläutert. Hierbei zeigen:
- Fig. 1: eine schematische Darstellung einer Anordnung für eine Vorrichtung zum dosierten Abgeben einer mikrofluidischen Menge eines Fluids, bei der in einer Ausbringleitung ein erstes und ein zweites Ventil in einer Reihenschaltung angeordnet sind;
- Fig. 2: eine schematische Darstellung einer Anordnung für eine Vorrichtung zum dosierten Abgeben einer mikrofluidischen Fluidmenge, bei der in der Ausbringleitung ein erstes und ein zweites Ventil in einer Parallelschaltung angeordnet sind; und
- Fig. 3: eine schematische Darstellung einer Anordnung für ein Handgerät zum lokalen Aufstechen einer menschlichen oder einer tierischen Haut.

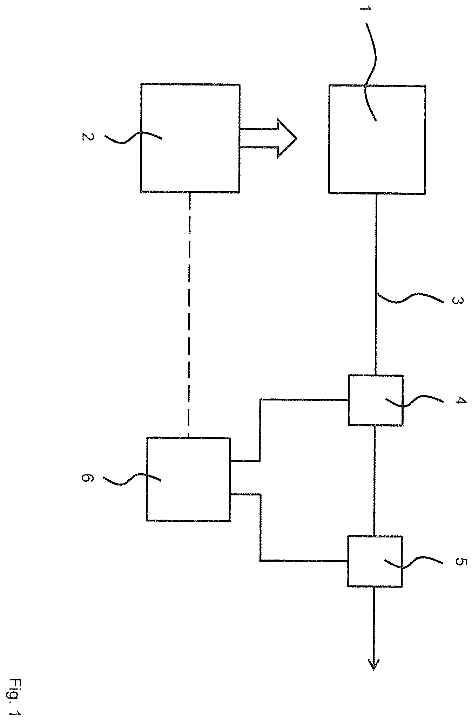

Fig. 1 zeigt eine schematische Darstellung einer Anordnung für eine Vorrichtung zum dosierten Abgeben oder Dosieren einer mikrofluidischen Fluidmenge, sei es im Rahmen einer Einzelpulsabgabe oder einer wiederholten Abgabe dosierter Fluidmengen mit einer (variierbaren) Wiederholfrequenz. In einem Fluidreservoir 1 ist eine Fluidmenge eines abzugebenden Fluids aufgenommen, zum Beispiel ein kosmetischer oder ein medizinischer Wirkstoff oder ein Farbstoff. Grundsätzlich können beliebige Fluide, insbesondere Flüssigkeiten, in dem Fluidreservoir 1 aufgenommen sein, um sie dosiert abzugeben.

Es ist eine Druckeinrichtung 2 vorgesehen, mit der das abzugebende Fluid mit Druck beaufschlagt wird, so dass das Fluid durch eine Ausbringleitung 3, die an das Fluidreservoir 1 anschließt, dosiert ausgestoßen werden kann. Beispielsweise weist die Druckeinrichtung 2 eine Pumpe auf.

In der Ausbringleitung 3 sind ein erstes Ventil 4 und ein zweites Ventil 5 hintereinander, einer Reihenschaltung entsprechend, angeordnet. Das erste Ventil 4 ist vom Typ Schließer, welches in einer Ruhestellung offen ist, um die Ausbringleitung 3 für einen Fluidstrom freizugeben, und in einer Arbeitsstellung geschlossen ist, um die Ausbringleitung 3 für den Fluidstrom zu schließen. Das zweite Ventil 5 ist vom Typ Öffner, welches in einer Ruhestellung geschlossen ist, um die Ausbringleitung 3 für den Fluidstrom zu schließen, und in einer Arbeitsstellung offen ist, um die Ausbringleitung 3 für den Fluidstrom freizugeben. Das erste Ventil 4 ist einer Ventilcharakteristik entsprechend mit einer minimalen Schließzeit ausgestattet. Das zweite Ventil 5 ist seiner Ventilcharakteristik entsprechend mit einer minimalen Öffnungszeit ausgebildet.

Es ist eine Steuereinrichtung 6 vorgesehen, die mit dem ersten und dem zweiten Ventil 4, 5 verbunden ist. Wahlweise kann die Steuereinrichtung 6 zusätzlich mit der Druckeinrichtung 2 verbunden sein, um deren Betrieb zu steuern, zum Beispiel für eine zur Ventilbetätigung in zeitlicher Hinsicht koordinierte Druckbeaufschlagung.

Mithilfe der Steuereinrichtung 6 werden erste Steuersignale zum Betätigen des ersten Ventils 4 und zweite Steuersignale zum Betätigen des zweiten Ventils 5 bereitgestellt. Mithilfe der ersten und der zweiten Steuersignale werden das erste und das zweite Ventil 4, 5 im Betrieb kontrolliert geöffnet und geschlossen. Die ersten und die zweiten Steuersignale können von der Steuereinrichtung 6 so bereitgestellt werden, dass ein oder mehrere Betriebsmodi zum dosierten Abgegeben einer Fluidmenge durch die Ausbringleitung 3 realisiert werden. Hierzu gehört das Öffnen der Ausbringleitung 3 für eine verkürzte minimale Öffnungszeit zum Freigeben der Ausbringleitung 3 für den Fluidstrom, wobei die verkürzte minimale Öffnungszeit kürzer als die minimale Öffnungszeit des zweiten Ventils 5 und wahlweise zusätzlich kürzer als die minimale Schließzeit des ersten Ventils 4 ist. Mittels Betätigens des ersten und zweiten Ventils 4, 5 ist es so ermöglicht, in zeitlicher Hinsicht kürzere Fluidimpulse auszustoßen oder abzugeben als dies mittels Betätigens nur des zweiten Ventils 5 vom Typ Öffner möglich wäre. Alternativ oder ergänzend kann eine Betriebsart vorgesehen sein, bei der die Ausbringleitung mittels Betätigens des ersten und des zweiten Ventils 4, 5 für einer verkürzte minimale Schließzeit geschlossen wird, die kürzer als die minimale Schließzeit des ersten Ventils 4 ist.

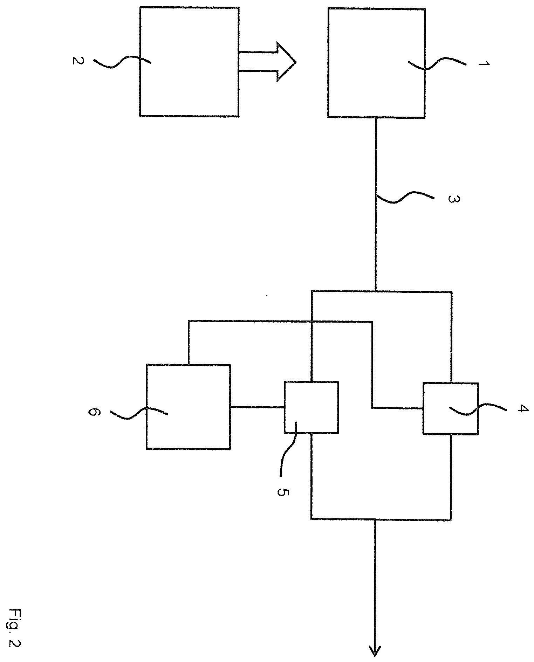

Fig. 2 zeigt eine schematische Darstellung einer weiteren Anordnung für eine Vorrichtung zum dosierten Abgeben einer Fluidmenge, bei der das erste und das zweite Ventil 4, 5 im Unterschied zur Ausführungsform in Fig. 1 nun in einer Parallelschaltung angeordnet sind. Bezüglich des Erzeugens der ersten und der zweiten Steuersignale mittels der Steuereinrichtung 6 sowie der hiermit realisierbaren Betriebsmodi für die Ausbringleitung 3 gelten die vorangehend in Verbindung mit der Ausführungsform in Fig. 1 gemachten Erläuterungen entsprechend. Das mittels der Steuereinrichtung 6 koordinierte und aufeinander abgestimmte Betätigen des ersten und des zweiten Ventils 4, 5 ermöglicht ein variables Öffnen und Schließen der Ausbringleitung 3, wobei insbesondere ein verkürztes Öffnen / Schließen der Ausbringleitung 3 im Vergleich zur minimalen Schließ- / Öffnungszeit der Einzelventile vorgesehen sein kann.

Die vorangehend und Bezugnahme auf die Fig. 1 und 2 erläuterten Anordnungen können zum Beispiel bei einem Handgerät zum lokalen Aufstechen einer menschlichen oder einer tierischen Haut verwendet werden, um bei einem solchen Stechgerät dosierte Fluidmengen eines in die Haut einzubringenden Stoffes in dosierten Mengen bereitzustellen.

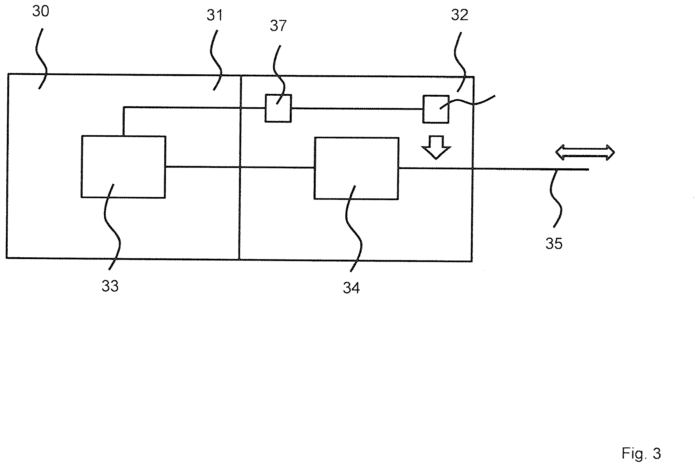

Fig. 3 zeigt eine schematische Darstellung einer Anordnung für ein solches Handgerät 30. Das Handgerät 30 verfügt über ein Antriebsmodul 31 und ein hieran gekoppeltes Stechmodul 32, sei es lösbar oder nicht lösbar koppelnd. Mithilfe einer Antriebseinrichtung 33 im Antriebsmodul 31 wird eine nicht-manuell (maschinell) erzeugte Antriebskraft oder -bewegung für eine im Stechmodul 32 angeordnete Stecheinrichtung 34 bereitgestellt, beispielsweise unter Verwendung eines Elektromotors. Das Stechmodul 32 kann ein Einwegmodul sein. Ausführungsformen für solche Handgeräte zum lokalen Aufstechen einer Haut sind als solche in verschiedene Ausführungsformen bekannt.

Mithilfe der Antriebskraft wird eine Stechnadel 35 der Stecheinrichtung 34, die mit einer Einzelnadel oder einer Nadelgruppe gebildet sein kann, aus- und eingefahren, sei es in einem Einzelstechmodus oder wiederholt entsprechend einer Wiederholfrequenz. Hierdurch kann eine menschliche oder eine tierische Haut lokal aufgestochen werden. Zeitlich koordiniert zu der Stechbewegung wird mithilfe einer Dosiereinrichtung 36 eine dosierte Fluidmenge bereitgestellt, um zum Beispiel einen kosmetischen oder einen medizinischen Wirkstoff bereitzustellen, der über die aufgestochene Haut eingebracht werden kann. Die Dosiereinrichtung 36 umfasst eine Anordnung in der Ausgestaltung nach Fig. 1 oder Fig. 2. Mittels einer Steuereinrichtung 37 des Handgeräts 30 können die Dosiereinrichtung 36 wie auch die Stechbewegung gesteuert werden, insbesondere zeitlich koordiniert zueinander.

Alternativ kann eine manuell betätigbare Stecheinrichtung vorgesehen sein. Um koordiniert zur manuell ausgelösten Stechbewegung die Abgabe oder Dosierung der Fluidmenge zu steuern, wird mittels einer Sensoreinrichtung die Stechbewegung detektiert. In Abhängigkeit hiervon wird dann die Dosierung der mikrofluidischen Menge des Fluids bewirkt, insbesondere mittels Betätigens der Dosiereinrichtung 36.

Die in der vorstehenden Beschreibung, den Ansprüchen sowie der Zeichnung offenbarten Merkmale können sowohl einzeln als auch in beliebiger Kombination für die Verwirklichung der verschiedenen Ausführungen von Bedeutung sein.

## Patentansprüche

1. Vorrichtung zum dosierten Abgeben einer mikrofluidischen Fluidmenge im Piko- und Mikroliterbereich, mit:
- einem Reservoir (1), welches eingerichtet ist, ein Fluid aufzunehmen;
- einer Ausbringleitung (3), die mit dem Reservoir (1) in Fluidverbindung steht und durch eine Fluidmengen des Fluids aus dem Reservoir (1) dosiert ausgebracht werden kann;
- einer Druckeinrichtung (2), die eingerichtet ist, das Fluid mit einem Ausbringdruck zu beaufschlagen, derart, dass die Fluidmengen über die Ausbringleitung (3) ausgestoßen werden;
- einer Ventileinrichtung aufweisend
- ein erstes Ventil (4) vom Typ Schließer in der Ausbringleitung (3), welches in einer Ruhestellung offen ist, um die Ausbringleitung (3) für den Fluidstrom freizugeben, und in einer Arbeitsstellung geschlossen ist, um die Ausbringleitung (3) für einen Fluidstrom zu schließen, wobei das erste Ventil (4) als Ventilcharakteristik eine minimale Schließzeit aufweist; und
- ein zweites Ventil (5) vom Typ Öffner in der Ausbringleitung (3), welches in einer Ruhestellung geschlossen ist, um die Ausbringleitung (3) für den Fluidstrom zu schließen, und in einer Arbeitsstellung offen ist, um die Ausbringleitung (3) für den Fluidstrom freizugeben, wobei das zweite Ventil (5) als Ventilcharakteristik eine minimale Öffnungszeit aufweist; und
- einer Steuereinrichtung (6), welche mit dem ersten und dem zweiten Ventil (4, 5) verbunden und eingerichtet ist, erste Steuersignale zum Betätigen des ersten Ventils (4) und zweite Steuersignale zum Betätigen des zweiten Ventils (5) bereitzustellen, derart, dass mittels Betätigen des ersten und des zweiten Ventils (4, 5) für die Ausbringleitung (3) wenigstens einer der folgenden Betriebsmodi zum dosierten Abgeben der Fluidmenge ausführbar ist:
- eine verkürzte minimale Öffnungszeit zum Freigeben der Ausbringleitung (3) für den Fluidstrom, welche kürzer als die minimale Öffnungszeit des zweiten Ventils (5) ist; und
- eine verkürzte minimale Schließzeit zum Schließen der Ausbringleitung (3) für den Fluidstrom, welche kürzer als die minimale Schließzeit des ersten Ventils (4) ist.

2. Vorrichtung nach Anspruch 1, **dadurch gekennzeichnet, dass** die Steuereinrichtung (6) weiterhin eingerichtet ist, die ersten Steuersignale zum Betätigen des ersten Ventils (4) und die zweiten Steuersignale zum Betätigen des zweiten Ventils (5) derart bereitzustellen, dass die verkürzte minimale Öffnungszeit zum Freigeben der Ausbringleitung (3) für den Fluidstrom kürzer als die minimale Schließzeit des ersten Ventils (4) ist.

3. Vorrichtung nach Anspruch 1 oder 2, **dadurch gekennzeichnet, dass** die Steuereinrichtung (6) weiterhin eingerichtet ist, die ersten Steuersignale zum Betätigen des ersten Ventils (4) und die zweiten Steuersignale zum Betätigen des zweiten Ventils (5) derart bereitzustellen, dass die verkürzte minimale Schließzeit zum Schließen der Ausbringleitung (3) für den Fluidstrom kürzer als die minimale Öffnungszeit des zweiten Ventils (5) ist.

4. Vorrichtung nach mindestens einem der vorangehenden Ansprüche, **dadurch gekennzeichnet, dass** die ersten und die zweiten Steuersignale eingerichtet sind, wenigstens einen der folgenden Betriebsmodi mit einer Wiederholfrequenz bereitzustellen:
- wiederholtes Öffnen der Ausbringleitung (3) für den Fluidstrom mit der verkürzten minimalen Öffnungszeit; und
- wiederholtes Schließen der Ausbringleitung (3) für den Fluidstrom mit der verkürzten minimalen Schließzeit.

5. Vorrichtung nach mindestens einem der vorangehenden Ansprüche, **dadurch gekennzeichnet, dass** das erste Ventil (4) und das zweite Ventil (5) in der Ausbringleitung (3) einer Ventilreihenschaltung entsprechend angeordnet sind.

6. Vorrichtung nach mindestens einem der vorangehenden Ansprüche, **dadurch gekennzeichnet, dass** das erste Ventil (4) und das zweite Ventil (5) in der Ausbringleitung (3) einer Ventilparallelschaltung entsprechend angeordnet sind.

7. Verfahren zum dosierten Abgeben einer mikrofluidischen Fluidmenge im Piko- und Mikroliterbereich, mit:
- Bereitstellen eines abzugebenden Fluids in einem Reservoir (1);
- einer Ausbringleitung (3), die mit dem Reservoir (1) in Fluidverbindung steht und durch die eine Fluidmenge des Fluids aus dem Reservoir (1) dosiert ausgebracht werden kann;
- Beaufschlagen des Fluids mit einem Ausbringdruck mittels einer Druckeinrichtung (2), derart, dass die Fluidmenge über die Ausbringleitung (3) ausgestoßen wird; und
- Steuern des Ausstoßens der Fluidmenge mittels einer Ventileinrichtung und einer hiermit verbundenen Steuereinrichtung (6), die Ventileinrichtung aufweisend
- ein erstes Ventil (4) vom Typ Schließer in der Ausbringleitung (3), welches in Ruhestellung offen ist, um die Ausbringleitung (3) für einen Fluidstrom freizugeben, und in einer Arbeitsstellung geschlossen ist, um die Ausbringleitung (3) für einen Fluidstrom zu schließen, wobei das erste Ventil (4) als Ventilcharakteristik eine minimale Schließzeit aufweist; und
- ein zweites Ventil (5) vom Typ Öffner in der Ausbringleitung (3), welches in Ruhestellung geschlossen ist, um die Ausbringleitung (3) für den Fluidstrom zu schließen, und in einer Arbeitsstellung offen ist, um die Ausbringleitung (3) für den Fluidstrom freizugeben, wobei das zweite Ventil (5) als Ventilcharakteristik eine minimale Öffnungszeit aufweist;
wobei die Steuereinrichtung (6) erste Steuersignale zum Betätigen des ersten Ventils (4) und zweite Steuersignale zum Betätigen des zweiten Ventils (5) bereitstellt, derart, dass mittels Betätigen des ersten und des zweiten Ventils (4, 5) für die Ausbringleitung (3) wenigstens einer der folgenden Betriebsmodi zum dosierten Abgeben der Fluidmenge ausgeführt wird:
- eine verkürzte minimale Öffnungszeit zum Freigeben der Ausbringleitung (3) für den Fluidstrom, welche kürzer als die minimale Öffnungszeit des zweiten Ventils (5) ist; und
- eine verkürzte minimale Schließzeit zum Schließen der Ausbringleitung (3) für den Fluidstrom, welche kürzer als die minimale Schließzeit des ersten Ventils (4) ist.

8. Handgerät zum lokalen Aufstechen einer menschlichen oder einer tierischen Haut, mit einer Vorrichtung nach mindestens einem der Ansprüche 1 bis 7.
